# EUROPÄISCHE PATENTSCHRIFT

(11) **EP 0 621 336 B1**
(45) Veröffentlichungstag und Bekanntmachung des Hinweises auf die Patenterteilung: **18.12.1996**
(21) Anmeldenummer: 94810121.7
(22) Anmeldetag: 28.02.1994
(51) Int. Cl.: C12M 1/36, C12P 5/02, C12M 1/113, C12M 1/107

(54) **Steuerung einer Vergärungsanlage**
Control of a fermentation plant
Commande d'une installation de fermentation

(30) Priorität: 21.04.1993 CH 1197/93
(43) Veröffentlichungstag der Anmeldung: 26.10.1994
(73) Patentinhaber: Schmid, Walter, CH-8152 Opfikon (CH)
(72) Erfinder: Schmid, Walter, CH-8152 Opfikon (CH)
(74) Vertreter: Feldmann, Clarence Paul

(56) Entgegenhaltungen:
- EP-A- 0 241 357
- WO-A-84/01363
- FR-A- 2 687 166
- GB-A- 2 204 056

## Beschreibung

Die vorliegende Erfindung betrifft eine Steuerung einer Vergärungsanlage, bei der biogene, zu vergärende Abfälle zerhackt und vorerwärmt einem Fermenter zugeführt werden, in dem der biogene Abbau mittels Methanbakterien erfolgt, wobei die frischen, zu vergärenden biogenen Abfälle eingangs des anaeroben Fermenters gesteuert mit weitgehend fermentierten, mit Methanbakterien angereicherten biogenen Abfällen vom Ausgang des Fermentes zusätzlich beimpft werden, und im Fermenter die biogenen Abfälle im Pfropfstrombetrieb mit kontrollierter Durchmischung unter Bildung von Biogas abgebaut und anschliessend einer aeroben Verrottung zugeführt werden.

Es sind zwei Typen von Arbeitsweisen bei der Vergärung von biogenen Abfällen bekannt, nämlich das verbreitete, chargenweise arbeitende Verfahren, mit vertikal stehenden Behälter welches äusserst langsam, aber gut beherrschbar ist, und das noch weniger verbreitete Verfahren, welches pfropfströmig und kontinuierlich mit liegendem Fermenter arbeitet, das aber schwierig kontrollier- und steuerbar ist.

Bei den charchenweise arbeitenden Verfahren werden üblicherweise eine oder mehrere stehende, vertikale Fermenter eingesetzt. Der Feinheitsgrad des zerkleinerten biogenen Abfalls ist nicht von besonderer Bedeutung, sondern beeinflusst lediglich die Fermentationsdauer. Die biogen abgebauten Abfallanteile sacken ab in Richtung zum Ausgang des Fermenters. Nur im Ausgangsbereich werden Proben entnommen und der Zeitpunkt bestimmt, an dem eine Charge abgelassen und neues Frischgut zugeführt wird. Problematisch ist hier weniger die Steuerung als vielmehr, dass schwerere Anteile schneller absacken und folglich unvollständig abgebaut in den Ausgangsbereich gelangen. Dies ist um so bedenklicher, als hierin oftmals auch Krankheitserreger enthalten sind, die wegen des zu geringen Aufenthaltes im Fermenter nicht abgetötet werden.

Um dies entgegenzuwirken, baut man heute vermehrt Vergärungsanlagen, die mit geneigten Fermentern arbeiten. Entsprechend dem Neigungsgrad fliesst das Material entsprechend langsamer. Ein schnelles Durchsacken schwerer Anteile wird somit weitgehend verhindert.

Bedingung aller bekannten anaeroben Fermenter in Vergärungsanlagen ist aber immer noch ein relativ gut fliessfähiges Gemisch im Fermenter, um eine hohe Homogenität im Fermenter zu erreichen, weil keine Korrekturmöglichkeiten vorgesehen sind.

So arbeitet eine Anlage gemäss der DE-A-32'28'895 mit einem geneigten Fermenter, der selber drehbar gelagert ist, der mit einer Mischung aus Klärschlamm und Müll arbeitet. Dies ergibt einen relativ gut fliessfähigen Inhalt, dessen Durchsatzgeschwindigkeit sich ausgangsseitig steuern lässt und zudem durch den Neigungswinkel beeinflussbar ist. Lediglich das einzufüllende Material kann vorgängig untersucht werden und dessen pH-Wert durch Zugabe von Kalk eingestellt werden.

Beim Fermenter gemäss der DE-A-25'35'756 wird hingegen mit dünnflüssigem Material gearbeitet, und ein leistungsfähiges Rührwerk verhindert die Bildung einer Schwimmdecke. Hier wird somit der gesamte Inhalt vermischt, was aber unproblematisch ist, weil chargenweise gearbeitet wird.

Aber auch bei vertikalen Fermentern wird das Füllmaterial möglichst fliessfähig gehalten, um homogene Verhältnisse im Fermenter zu erreichen. So wird in der EP-131'319 vorgeschlagen, jeweils den Fermenter mit einen Teil Frischgut und zwei Teile bereits abgebauter Materie zu beschicken. In der DE-A-29'24'387 hingegen wird das Füllgut bis zu pumpfähigem Material verfeinert.

Obwohl in verschiedenen Patenten mit geneigten Fermentern von Pfropfstrombetrieb gesprochen wird, so handelt es sich doch nur um quasi Pfropfstrombetrieb, der schubweise erfolgt.

Auch das in der EP-A-0'241'357 offenbarte Verfahren arbeitet mit einem liegenden Fermenter, der im Quasi-Pfropfstrombetrieb chargenweise arbeitet und über eine zentrale Steuereinheit steuerbar ist. Hierbei wird der Inhalt des Fermenters an einer Stelle bezüglich Verunreinigungen überwacht. Mittels einer Pumpe wird dem eingespiesenen Cellulosesubstrat vorgereinigtes Abwasser zugeführt. Ueberschüssige Flüssigkeit wird über einen Ueberlauf und einer Rückleitung in den Fermenter zurückgeführt.

In einem Flüssigkeitsfermenter gemäss der GB-A-2'204'056 wird das durch anaerobe Fermentation gewonnene Gas teilweise in den Fermenter zurückgeführt, um das flüssige Substrat aufzuwirbeln.

Einen Mehrkammerfermenter zeigt die WO-A-84/01363. Von einem Speisebehältnis kann abzubauendes Substrat in die verschiedenen Fermenterkammern und von diesen untereinander weitergeleitet und schliesslich abgeleitet werden. Der Ablauf kann mittels Sensoren an verschiedenen Stellen überwacht und gesteuert werden. Hierbei wird die Temperatur, der pH-Wert und der Druck gemessen und überwacht.

Schliesslich zeigt die FR-A-2'687'166 einen Fermenter, der computergesteuert arbeitet. Ein Teil des chargenweise arbeitenden Fermenters gelangt in einen Experimentierfermenter, in dem im Kleinen der Ablauf simuliert und beeinflusst werden kann, um danach den Hauptfermenter entsprechend zu steuern.

Das erfindungsgemässe Verfahren arbeitet mit einem vollständig horizontal liegenden Fermenter gemäss der EP-A-476'217, der im echten, das heisst kontinuierlichen, Pfropfstrombetrieb arbeitet, der somit über seiner gesamten Länge vom Eingang bis zum Ausgang sich kontinuierlich variierende Verhältnisse aufweist.

Aufgabe der Erfindung ist es nun, ein Verfahren anzugeben, welches eine einwandfreie Steuerung eines solchen Fermenters in einer Vergärungsanlage gemäss Oberbegriff des Patentanspruches ermöglicht, so dass ein absolut sicherer kontinuierlicher Betrieb möglich ist.

Diese Aufgabe erfüllt das Verfahren gemäss Patentanspruch 1. Das Verfahren geht von der Erkenntnis aus, dass in einem echten Pfropfstrombetrieb das Füllmaterial unterschiedlich ist und somit auch sich unterschiedlich abbaut, was während des Durchstromes des Füllmaterials entsprechende Korrekturen ermöglichen muss.
Zudem geht man von der biologischen Erkenntnis aus, dass der pH-Wert in direkter Relation steht zu der Menge vorhandenen und aktiven Methanbakterien, wobei man diese nicht nur am Eingang zugeben kann, wenn man gemäss Patentanspruch 2 verfährt.

Da nun der Fermenterinhalt nicht mehr in der Längsachse durchmischbar ist, wird nicht gerührt, sondern lediglich schrittweise das Material umgewälzt, ohne dass ein Vorschub entsteht, so dass keine Durchmengung stattfindet, sondern lediglich eine Umwälzung, die ein Entgasen des Füllmaterials begünstigt.

Die Verwendung von Presswasser dient nicht nur der Regelung der gewünschten feuchten Atmosphäre, sondern führt gleichzeitig Methanbakterien zu. Erfolgt dies an verschiedenen Stellen im Fermenter, so lässt sich durch die Zuführung von Methanbakterien auch der pH-Wert beeinflussen und somit steuern.

Insbesondere die Zuleitung von mit Methanbakterien beladenen Presswasser in die Förder- und Mischstrecke der Speiseleitung erlaubt das einströmende Material, vorwiegend Frischgut, optimal auf den gewünschten pH-Wert bereits eingangs des Fermenters einzustellen, wodurch nachträglich Korrektureingriffe weitgehend vermieden werden.

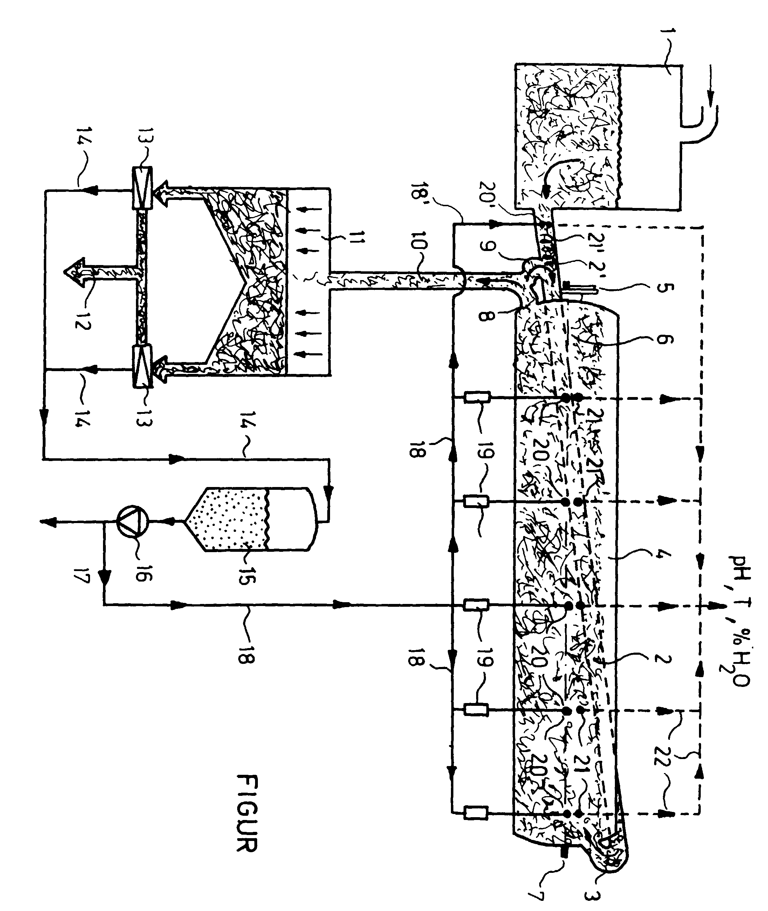

Da die Abbaugeschwindigkeit auch temperaturabhängig ist, wird man vorteilhafterweise das einzuleitende Presswasser annähernd auf die Temperatur vorheizen, welche am Einleitungsort herrscht. In der einzigen Figur ist eine schematische Darstellung eines Teiles einer Vergärungsanlage gezeigt, anhand dessen das efindungsgemässe Verfahren erläutert wird.

Die angelieferten biogenen Abfälle, es handelt sich hierbei um getrennt eingesammelten Hausmüll, Gartenabfälle, sowie Abfälle von gewerblichen Betrieben mit grossem Anteil an biogenen Abfällen, wie Gärtnereien, Gemüsehandel, Restaurants usw., werden in einen Annahmebunker zwischengelagert. Von dort, gelangen sie über eine Sortieranlage und einer Zerkleinerungsvorrichtung schliesslich in einen Speisebunker 1, von dem aus das biogene, zu vergärende Material dosiert über eine Speiseleitung 2 zum Eingang 3 eines anaeroben geheizten Fermenter 4 gelangt.Im Speisebunker findet eine Versäuerung statt, so dass sich beim Frischgut ein pH-Wert von cirka 5-6 einstellt. Dies kann auch durch eine zusätzliche Hydrolyseanlage begünstigt werden. In der Speiseleitung 2 ist eine Misch- und Förderstrecke 2' vorgesehen. diese arbeitet vorzugsweise mit einer Förder- und Mischschnecke. Ueber einen Antrieb 5 werden mehrere Rechenarme, die auf einer zentralen Welle 6 befestigt sind, schrittweise bewegt. Die zentrale Welle 6 durchsetzt den Fermenter auf der ganzen Länge und ist auf der Gegenseite bei 7 gegengelagert. Das im Pfropfstrombetrieb kontinuierlich, durch das eingangseitig nachgeschobene Material, sich vorwärtsbewegende, zu vergärende Gut gelangt zum Fermenterausgang 8, wo über eine Rückkopplung 9 bereits weitgehend abgebaute biogene Abfälle dem Frischgut vom Speisebunker in die Speiseleitung 2 gesteuert beigegeben und vermischt werden kann. Das weitgehend biologisch abgebaute Material wird danach über eine Leitung 10 zu einer Presse 11 geleitet, wo das bereits fermentierte Material unter Druck entwässert wird. Durch eine Förderleitung 12 gelangt schliesslich das weitgehend biologisch abgebaute Material zu einer nicht dargestellten aeroben Nachrotte. Das vollständig abgebaute Material gelangt schliesslich als hochwertige Komposterde in den Handel.

Ueber die Trennfilter 13 werden die Flüssiganteile ausgangs der Presse 11 abgetrennt. Dieser Flüssiganteil, das Presswasser, ist bakteriell stark beladen, vorwiegend mit Methanbakterien. Das Presswasser gelangt über Rohrleitungen 14 zu einem Presswassertank 15. Mittels einer Pumpe 16 wird das überschüssige Presswasser zu einer nicht dargestellten Presswasseraufbereitungsanlage 17 gefördert. Hier wird das Presswasser mittels einem speziellen anaeroben Filtersystem weiter abgebaut unter Produktion von weiterem Biogas.

Ein anderer Teil des Presswassers gelangt über Steuerleitungen 18 zu verschiedenen Impfstellen 20 des Fermenters 4. Im Bereich der Impfstellen 20 befinden sich auch Messtellen 21. An diesen Stellen lassen sich die pH-Wert, die Temperatur und der Feuchtigkeitsgehalt des Fermenterinhaltes in jeder Region ermitteln. Ueber Datenleitungen 22 ist es möglich, diese Daten einer zentralen Auswerteeinheit zuzuführen.

Ein weiterer Zweig 18' der Steuerleitungen 18 gelangt in den Bereich der Förder- und Mischstrecke 2' der Speiseleitung 2. Auch hier ist eine Impfstelle 20' und eine Messtelle 21' angeordnet. Dies erlaubt bereits das Frischgut mittels Presswasser bakteriell zu beladen und auf einen optimalen pH-Wert einzustellen. In je engeren Grenzen der pH-Wert bereits eingangsseitig beim Fermenter 4 anliegt. umso weniger Stereingriffe sind folglich nötig. Auch die Messdaten in der Misch- und Förderstrecke 2' wird man über die Datenleitung 22 der zentralen Auswerteeinheit zuführen.

Nachfolgend wird nun das erfindungsgemässe Verfahren im Ablauf beschrieben. Das im Speisebunker 1 anliegende Frischgut aus biogenen Abfällen, das bereits von nicht biologisch abbaubaren Materialien, wie Glas, Metall und Kunststoffanteilen, getrennt ist und auch bereits zerkleinert ist, ist in der Zusammensetzung nicht beeinflussbar. Es kommt über die Speiseleitung 2 zum Eingang 3 des anaeroben Fermenters 4. Bereits in der Speiseleitung 2 wird das Frischgut vorgewärmt auf eine Temperatur, die für die Vermehrung der Methanbakterien besonders günstig ist, vorzugsweise auf Temperaturen zwischen 30° und 60° C. Um das Frischgut mit bakteriell bereits angereichertem Material anzureichern, wird über eine Rückkupplung 9, in an sich bekannter Weise, bereits weitgehend biologisch abgebautes Material vom Ausgang 8 des Fermenters 4 in die Speiseleitung 2 eingeimpft. Dieses eingangs des Fermenters 4 anliegende Material ist relativ trocken, und folglich mit einem hohen Trockensubstanzanteil und relativ grob, mit Partikelgrössen bis zu cirka 10 cm. Entsprechend strömt das Material im Fermenter nicht selbstständig. Es lässt sich lediglich mittels nachgepresstem Material durch den Fermenter in echten Pfropfstrombetrieb hindurchpressen. Eingangs des Fermenters 4 wird an einer ersten Messtelle 21 der pH-Wert, die Temperatur und der Feuchtigkeitsgehalt ermittelt. Uebliche Messwerte liegen an dieser Stelle bei pH 7,2 - 6,5, also im neutralen bis leicht sauren Bereich. Vorzugsweise wird man den Wert auf cirka pH 7 einstellen. Ausgangs des Fermenters liegt der pH-Wert circa auf 8,4 - 7,5, also im basischem Bereich, vorzugsweise bei cirka pH 8.

Zur Einstellung des pH-Wertes stehen verschiedene Massnahmen zur Verfügung. Im Eingangsbereich kann der pH-Wert durch das Mischverhältnis von Frischgut aus dem Speisebunker 1 und bereits weitgehend abgebauten Faulgut aus dem anaeroben Fermenter 4 über die Rückkopplung 9 entsprechend eingestellt werden. Hierdurch lässt sich durch die höhere Beigabe von Faulgut der pH-Wert anheben, beziehungsweise durch Reduktionen senken.

Eine weitere Beeinflussungsmöglichkeit besteht in der Senkung der Zufuhrmenge des Frischgutes. Hierdurch wird die Verweilzeit im Fermenter verlängert. Dies hat zur Folge, dass die Methanbakterien mehr Zeit zur Vermehrung haben insbesondere im nährstoffreichen Eingangsbereich, wodurch im Eingangsbereich ein leichter Anstieg des pH-Wertes erfolgt, gleichzeitig aber auch ausgangseitig der pH-Wert etwas stärker ansteigt als bei der üblichen Durchsatzgeschwindigkeit. Diese Massnahme wird man ergreifen, wenn der Feuchtigkeitsgehalt eingangsseitig bereits im oberen Bereich liegt.

Ist der Trockensubstanzanteil hoch und der pH-Wert eingangsseitig jedoch relativ tief, so kommt in diesem Bereich auch die Möglichkeit in Frage, über die Steuerleitung 18 bakteriell beladenes Presswasser aus dem Presswassertank 15 in den Fermenter einzuleiten. Dies erhöht die Dichte der Methanbakterien und erhöht die Feuchtigkeit, so dass der bakterielle Abbau beschleunigt wird.

Selbstverständlich erhöht die vermehrte Zufuhr von Material am Eingang die Durchlaufgeschwindigkeit durch den gesamten Fermenter überall in gleicher Weise. So kann folglich durch Erhöhung der Durchlaufgeschwindigkeit auch vermieden werden, dass der pH-Wert ausgangsseitig über einen wünschbaren Wert steigt.

Meist kann davon ausgegangen werden, dass der pH-Wert während des Durchlaufs des Materials durch den Fermenter kontinuierlich innerhalb einer zulässigen Bandbreite steigt. Gelegentlich können jedoch auch Abweichungen auftreten, vorwiegend durch vorzeitiges Absterben der Methanbakterien. Dies führt dann zu einer Veränderung des pH-Wertes. Im erfindungsgemässen Verfahren wird daher an mehreren Stellen der pH-Wert ermittelt und direkt an der entsprechenden Stelle eingegriffen. Dies erfolgt wiederum durch die Beigabe von Presswasser durch die Steuerleitungen 18.

Vorzugsweise wird man versuchen den pH-Wert des Beschickungsmaterials, also des Frischgutes, beziehungsweise das Gemisch aus Frischgut aud dem Speisebunker 1 und des Faulgutes vom Ausgang des Fermentes, bereits in möglichst engen Grenzen zu halten. Hierdurch reduzieren sich die Anzahl der Korrektureingriffe. Erfindungsgemäss wird folglich bereits in der Förder- und Mischstrecke 2' der Speiseleitung 2 Presswasser zugeführt. Dieses Gelangt über den Zweig 18' der Steuerleitung 18 Impfstelle 20', die sich direkt neben einer Messtelle 21' befindet. Während das Frischgut eher sauer ist und das Presswasser aber basisch kaum eine Neutralisation erreicht werden, die für die Vermehrung der Methanbakterien vorteilhaft ist. Durch die Zufuhr von Frischwasser wäre dies nicht erreichbar oder müsste in sehr viel grösseren Mengen erfolgen, was aber bei der Erfindung vermieden werden soll.

Schliesslich wird auch der Temperaturverlauf im Fermenter überwacht. Damit durch Steuereingriffe in der Form von Zugabe von Presswasser die Temperatur nicht unzulässig absinkt, ist es vorteilhaft, die Steuerleitungen 18 mittels einem Heizelement 19 auf die dort im Fermenter herrschende Temperatur mindestens annähernd zu erwärmen. Das Heizelement kann ein elektrisches Heizelement oder auch ein Wärmetauscher sein.

Auch der Fermenter selbst, der durch Heizleitungen (nicht dargestellt) beheizt ist, kann segmentweisen beheizt sein. Dies erlaubt über die Change des Fermenters die Temperatur zu verändern, um die optimalen Wachstumsbedingungen der Methanbakterien zu erreichen.

Obwohl bei einer solchen Vergärungsanlage die Produktion von Biogas eine wesentliche Aufgabe ist, wird hierauf nicht weiter eingegangen. Selbstverständlich muss der Fermenter mit entsprechenden Auslasstellen für das im Fermenter entwickelte Biogas ausgerüstet sein. Die kontrollierte Führung des pH-Wertes und der Temperatur, so wie die optimale Einstellung des Feuchtigkeitsgehaltes führt zur grösstmöglichen Biogasproduktion, da die Lebensbedingungen für Methanbakterien optimal sind. Ein wesentlicher Punkt beim erfindungsgemässen Verfahren ist auch, dass das Material im anaeroben Fermenter 4 nicht durch einen Rührer vermischt wird, wie in den bekannten Verfahren, sondern lediglich langsam, schrittweise ungewäzlt wird. So wird lediglich erreicht, dass die freigesetzten Methan- und CO₂-Gase besser entweichen können. Hingegen wird auf diese Weise vermieden, dass noch kaum abgebautes Füllgut in den Ausgangsbereich des Fermenters gelangt.

Auch die für solche Verfahren ungewöhnlich trockene Atmosphäre, ohne einer durchgehenden Flüssigphase im Fermenter, verunmöglicht den Transport von Viren oder Bakterien über die Flüssigphase in kurzer Zeit vom Eingang zum Ausgang des Fermenters und somit eine Kontaminierung des entstehenden Kompostes.

Durch die optimale Konditionierung der Verhältnisse im Behälter wird eine für solche Vergärungsanlagen erstaunlich hohe Durchsatzgeschwindigkeit von cirka 20 Tage erreicht.

## Patentansprüche

1. Verfahren zur Steuerung einer Vergärungsanlage, bei der biogene, zu vergärende Abfälle zerhackt und vorerwärmt einem Fermenter zugeführt werden, in dem der biogene Abbau mittels Methanbakterien erfolgt, wobei die frischen zu vergärenden biogenen Abfälle eingangs des anaeroben Fermenters gesteuert mit weitgehend fermentierten, mit Methanbakterien angereicherten biogenen Abfällen vom Ausgang des Fermenters zusätzlich beimpft werden, und im Fermenter die biogenen Abfälle im Pfropfstrombetrieb mit kontrollierter Durchmischung unter Bildung von Biogas abgebaut und anschliessend einer aeroben Verrottung zugeführt werden, dadurch gekennzeichnet, dass der methanbakterielle Abbau während einer horizontalen Pfropfstrombewegung an mehreren Stellen des Förderweges im Fermenter mindestens bezüglich des pH-Wertes und der Trockensubstanzanteile überwacht und eingestellt wird und die Durchsatzgeschwindigkeit in Abhängigkeit dieser Messwerte einerseits durch die Zufuhr von zu vergärenden biogenen Abfällen und andererseits durch die Rückführung von weitgehend fermentierten biogenen Abfällen gesteuert wird.

2. Verfahren nach Anspruch 1, dadurch gekennzeichnet, dass die teilweise abgebauten biogenen Abfälle vor der Einleitung in die aerobe Verrottung gepresst werden, worauf durch Zufuhr des bakteriell beladenen Presswassers ein gewünschter Trokkensubstanz-Sollwert und ein gewünschter pH-Wert im anaeroben Fermenter eingestellt wird.

3. Verfahren nach Anspruch 2, dadurch gekennzeichnet, dass der pH-Wert und der Trockensubstanzanteil bereits im Bereich einer Förder- und Mischstrecke (2') in der Speiseleitung (2) an einer Messtelle (21') erfasst und entsprechend durch Zufuhr (18') von mit Methanbakterien beladenen Presswasser eingestellt wird.

4. Verfahren nach Anspruch 1, dadurch gekennzeichent, dass der Inhalt des anaeroben Fermenters schrittweise vorschubslos umgewälzt wird.

5. Verfahren nach Anspruch 2 und 3, dadurch gekennzeichnet, dass das Presswasser im Bereich der verschiedenen Messstellen (20) in den Fermenter (4) eingeleitet wird.

6. Verfahren nach Anspruch 2 und 5, dadurch gekennzeichnet, dass das Presswasser vor der Einleitung an der entsprechenden Stelle im Fermenter annähernd auf die dort herschende Temperatur aufgeheizt wird.

7. Verfahren nach Anspruch 1, dadurch gekennzeichnet, dass an den Messtellen (20) auch die dort herrschende Temperatur gemessen wird.

8. Verfahren nach Anspruch 7, dadurch gekennzeichnet, dass der Fermenter (4) in Längsrichtung segmentweise steuerbar beheizt wird.

## Claims

1. Process for the control of a fermentation plant, in which the biogenic waste to be fermented is chopped up and preheated and supplied to a fermenter, where biogenic decomposition takes place by means of methane bacteria, the fresh, biogenic waste to be fermented at the inlet of the anaerobic fermenter being inoculated in controlled manner with the substantially fermented, methane bacteria-enriched, biogenic waste from the fermenter outlet and in the fermenter the biogenic waste is decomposed in plug flow operation with controlled intermixing and accompanied by the formation of biogas and is then supplied to an aerobic rotting process, characterized in that the methane bacterial decomposition is monitored and adjusted at several points of the feed path in the fermenter at least with respect to the pH-value and dry matter fractions and the flow rate is controlled as a function of these measured values on the one hand by the supply of biogenic waste to be fermented and on the other by the recycling of largely fermented, biogenic waste.

2. Process according to claim 1, characterized in that the partly decomposed, biogenic waste is compressed prior to introduction into the aerobic rotting process and then through the supply of the bacteria-containing compression water a desired dry matter desired value and a desired pH-value are set in the anaerobic fermenter.

3. Process according to claim 2, characterized in that the pH-value and dry matter fraction is determined in the feedline (2) in the vicinity of a feed and mixing section (2') at a measuring point (21') and correspondingly set by the supply (18') of methane bacteria-containing compression water.

4. Process according to claim 1, characterized in that the content of the anaerobic fermenter is circulated stepwise and without advance.

5. Process according to claim 2 and 3, characterized in that the compression water is introduced into the fermenter (4) in the vicinity of the different measuring points (20).

6. Process according to claim 2 and 5, characterized in that the compression water, prior to introduction at the corresponding point in the fermenter, is heated approximately to the temperature prevailing there.

7. Process according to claim 1, characterized in that at the measuring points (20) the temperature prevailing there is also measured.

8. Process according to claim 7, characterized in that the fermenter (4) is controllably heated segmentwise in the longitudinal direction.

## Revendications

1. Procédé de commande d'une installation de fermentation, dans lequel on achemine les déchets biogènes à faire fermenter haché et préchauffé dans un fermentateur, la décomposition biogène se faisant à l'aide de bactéries dégageant du méthane, les déchets biogènes frais à faire fermenter étant de plus inoculés de manière commandée à l'entrée du fermentateur anaérobie à l'aide de déchets biogènes fermentés dans une large mesure, enrichis à l'aide de bactéries dégageant du méthane en provenance de la sortie du fermentateur, les déchets biogènes dans le fermentateur étant décomposés en fonctionnement selon un écoulement par trop plein d'un mélange contrôlé avec formation de gaz de digestion et étant ensuite soumis à une décomposition aérobie, caractérisé en ce que la décomposition bactérienne avec formation de méthane est surveillée et réglée pendant l'écoulement par trop plein horizontal, en plusieurs endroits du trajet de transport dans le fermentateur, au moins en ce qui concerne la valeur du pH et les proportions de substances sèches, et en ce que la vitesse de passage est commandée en fonction de ces valeurs de mesure, d'une part grâce à l'apport de déchets biogènes à faire fermenter et, d'autre part grâce à la recirculation des déchets biogènes fermentés dans une large mesure.

2. Procédé selon la revendication 1, caractérisé en ce que les déchets biogènes partiellement décomposés sont comprimés avant d'être soumis à la décomposition aérobie, à la suite de quoi des valeurs de consigne souhaitées de substances sèches et de pH sont réglées dans le fermentateur anaérobie, grâce à l'apport d'eau sous pression chargée de manière bactérienne.

3. Procédé selon la revendication 2, caractérisé en ce que la valeur de pH et la proportion de substances sèches sont mesurées déjà dans le domaine d'un parcours de transport et de mélange (2') dans la conduite d'alimentation (2) en un point de mesure (21') et sont réglées en conséquence grâce à un apport (18') d'eau sous pression chargée de bactéries dégageant du méthane.

4. Procédé selon la revendication 1, caractérisé en ce que le contenu du fermentateur anaérobie est pulsé pas à pas, sans évacuation.

5. Procédé selon la revendication 2 et 3, caractérisé en ce que l'eau sous pression est introduite dans le fermentateur (4) dans la zone des divers points de mesure (20).

6. Procédé selon la revendication 2 et 5, caractérisé en ce que l'eau sous pression est préchauffée avant introduction au point correspondant dans le fermentateur, approximativement à la température qui y règne.

7. Procédé selon la revendication 1, caractérisé en ce que l'on relève également, aux points de mesure (20), la température qui y règne.

8. Procédé selon la revendication 7, caractérisé en ce que le fermentateur (4) est chauffé de manière commandée par zones dans la direction longitudinale.
